# EUROPEAN PATENT APPLICATION

(11) **EP 3 925 656 A2**
(43) Date of publication of application: **22.12.2021**
(21) Application number: 21176424.6
(22) Date of filing: 28.05.2021
(51) Int. Cl.: A61M 25/02

(54) **CATHETER SECUREMENT DEVICE**

(30) Priority: 28.05.2020 US 202063031193 P
(71) Applicant: Prytime Medical Devices, Inc., Boerne TX 78006 (US)
(72) Inventor: PICKERING, Mathew Charles, Boerne, 78006 (US); FRANKLIN, Curtis J., Colorado, 80228 (US); BURGETT, Julie A., Boerne, 78006 (US)
(74) Representative: Westphal, Mussgnug & Partner Patentanwälte mbB

(57) **Abstract**

A kit includes a catheter and a catheter securing device. The catheter has a catheter shaft. The catheter securing device includes a base surface selectively securable to an intended surface and at least one catheter aperture. The catheter shaft extends through the at least one catheter aperture, such that the catheter securing device is mounted on the catheter shaft. The catheter securing device also includes a channel for stabilizing the catheter shaft thereon and a fastener repeatedly movable between a first position and a second position, independently of securement of the base surface to the intended surface. In the first position, the fastener permits substantially free sliding of the catheter shaft through the at least one catheter aperture, and, in the second position, the fastener substantially secures the catheter shaft to the catheter securing device in a substantially non-sliding manner.

## Description

### CROSS-REFERENCE TO RELATED APPLICATION

This application claims priority from U.S. Provisional Patent Application No. 63/031,193, titled "Catheter Securement Device", filed on May 28, 2020, the entire contents of which are incorporated by reference herein.

### BACKGROUND OF THE DISCLOSURE

The disclosure relates to a catheter securement device.

Catheters are generally secured to a patient after insertion of the catheter into the patient at an access site and an occlusion balloon is guided into an appropriate vessel to a predetermined location. Catheter securement is important to assist in maintaining the catheter (and the occlusion balloon) in the appropriate position.

Conventional catheter securement devices are generally provided separately from the catheter and must be attached to the catheter at some point after the catheter is readied for use. One drawback of such securement devices is that they may become separated or lost. Other conventional catheter securement devices combine attachment of the securement device to the catheter with the securement mechanism to the patient. One drawback of such securement devices is that repositioning of the catheter requires unsecuring the device from the patient as well, which generally requires removal of sutures and removal of the entire securement device from the catheter.

It would, therefore, be advantageous to manufacture a catheter securement device that is pre-attached to the catheter and permits repositioning of the catheter without unsecuring the device from the catheter or patient.

### BRIEF SUMMARY OF THE DISCLOSURE

Briefly stated, one aspect of the present disclosure is directed to a kit having a catheter and a catheter securing device. The catheter has a catheter shaft. The catheter securing device includes a base surface selectively securable to an intended surface and at least one catheter aperture. The catheter shaft extends through the at least one catheter aperture, such that the catheter securing device is mounted on the catheter shaft. The catheter securing device also includes a channel for stabilizing the catheter shaft thereon and a fastener repeatedly movable between a first position and a second position, independently of securement of the base surface to the intended surface. In the first position, the fastener permits substantially free sliding of the catheter shaft through the at least one catheter aperture, and, in the second position, the fastener substantially secures the catheter shaft to the catheter securing device in a substantially non-sliding manner.

In one configuration, the fastener includes a deflectable post projecting from the base surface and a latch selectively movable relative to the base surface. In the first position, the latch is disengaged from the deflectable post, and in the second closed position, the latch is in snap engagement with the deflectable post, substantially securing the catheter shaft to the catheter securing device via at least one of a compressive or friction force of the latch onto the catheter shaft.

In any of the previous configuration, the deflectable post may be positioned on one side of the channel and the latch may be pivotably attached to the base surface at an opposing side of the channel, whereby, in the second position, the latch extends over the channel.

In any of the previous configurations, the latch may be pivotably attached to the base surface via a living hinge.

In any of the previous configurations, the deflectable post may be selectively and elastically deflectable to disengage the latch therefrom and return the latch from the second position into the first position thereof.

In any of the previous configurations, the channel may be a grooved channel formed in the base surface.

In any of the previous configurations, the catheter securement device may include at least one of a friction enhancing insert along the channel or a friction enhancing insert along an underside of the latch, configured to form an interference fit with the catheter shaft in the closed position of the latch.

In one configuration, the fastener includes a housing upon the base surface, the housing having an open side and a pair of oppositely disposed apertures formed in opposing peripheral sides of the housing. A plunger is slidably received within the housing via the open side thereof. The plunger defines the channel. A biasing member is positioned between the plunger and a surface opposing the open side of the housing, the biasing member being configured to provide an ejecting force biasing the plunger through the open side of the housing. The catheter shaft extends through the at least one catheter aperture, the pair of oppositely disposed apertures of the housing and the at least one catheter aperture, wherein, in the second position, the ejecting force misaligns the channel from the pair of oppositely disposed apertures of the housing and the at least one catheter aperture, thereby securing the catheter shaft between the channel and the pair of oppositely disposed apertures of the housing. In the first position, the channel is substantially aligned with the pair of oppositely disposed apertures of the housing and the at least catheter aperture, permitting substantially free sliding of the catheter shaft therethrough.

In the previous configuration, the ejecting force may be configured to automatically position the fastener in the second position thereof, the fastener being selectively movable to the first position thereof under application of a force opposing, and greater than, the ejecting force.

In one configuration, the fastener includes a housing upon the base surface having an externally threaded screw projecting therefrom, the housing and externally threaded screw defining the channel. A compressible member is positioned along the channel, the catheter shaft extending along the channel and through the compressible member. An internally threaded collar is selectively threadably advanceable and retractable upon the externally threaded screw. In the second position, the collar is threadedly advanced upon the externally threaded screw to a position radially compressing the compressible member upon the catheter shaft to substantially prevent sliding of the catheter shaft therethrough along the channel, and in the first position, the collar is threadedly retracted from the externally threaded screw relative to the second position to a position wherein the compressible member permits substantially free sliding of the catheter shaft therethrough and along the channel.

In any of the previous configurations, the catheter securement device may include at least one of a fastening aperture or a landing tab, the fastening aperture being positioned along the base surface and configured for receiving a fastening tool therethrough to secure the catheter securement device to the intended surface, and the landing tab including a neck portion extending laterally from the base surface and terminating in a transverse wing portion, the neck portion being narrowed relative to the wing portion and dimensioned to permit application of a staple thereupon.

In the previous configuration, the at least one fastening aperture may include two fastening apertures positioned on opposite sides of the channel.

In any of the previous configurations, the base surface may include a pair of oppositely disposed, laterally projecting winged portions, each of the winged portions having a fastening aperture therein configured for receiving a fastening tool therethrough to secure the catheter securement device to the intended surface.

In any of the previous configurations, the base surface may include at least one landing tab, the landing tab having a neck portion extending laterally from the base surface and terminating in a transverse wing portion, the neck portion being narrowed relative to the wing portion and dimensioned to permit application of a staple thereupon.

In the previous configuration, the at least one landing tab may include a pair of oppositely disposed landing tabs

In any of the previous configurations, at least a portion of the base surface may include a window-like lattice defined by a plurality of window frames forming windows therebetween, the window-like lattice being configured to selectively secure the catheter securing device to the intended surface via a fastening tool.

In any of the previous configurations, the catheter securement device may include a friction enhancing pad positioned upon the base surface, the channel being formed in the friction enhancing pad.

In any of the previous configurations, the catheter securement device may include a friction enhancing insert positioned along the channel.

In any of the previous configurations, the catheter securement device may include at least one upwardly projecting tab, each one of the at least one upwardly projecting tab including one of the at least one catheter aperture.

In the previous configuration, the at least one upwardly projecting tab may include two upwardly projecting tabs and the at least one catheter aperture may include two coaxial catheter apertures.

### BRIEF DESCRIPTION OF THE SEVERAL VIEWS OF THE DRAWINGS

The following description of embodiments of the disclosure will be better understood when read in conjunction with the appended drawings. It should be understood, however, that the disclosure is not limited to the precise arrangements and instrumentalities shown. In the drawings:
Fig. 1 is a perspective top, rear and side view of a catheter securement device according to a first embodiment of the present disclosure, with a fastening mechanism thereof in an open position;
Fig. 2 is a rear elevational view of the catheter securement device of Fig. 1, with the fastening mechanism thereof in the open position;
Fig. 3 is a perspective top, front and side view of the catheter securement device of Fig. 1 mounted onto a catheter, with the fastening mechanism thereof in the open position;
Fig. 4 is perspective top view of the catheter securement device of Fig. 1 mounted onto a catheter, with the fastening mechanism thereof in a closed position;
Fig. 5 is a perspective top, rear and side view of the catheter securement device of Fig 1, having inserts included to counter catheter sliding;
Fig. 6 is a perspective top, front and side view of a catheter securement device according to a second embodiment of the present disclosure, with a fastening mechanism thereof in an open position;
Fig. 7 is a perspective top, front and side view of the catheter securement device of Fig. 6, with the fastening mechanism thereof in a closed position;
Fig. 8 is a perspective top, front and side view of a catheter securement device according to a third embodiment of the present disclosure, with a fastening mechanism thereof in a closed position;
Fig. 9 is a perspective top, front and side view of a catheter securement device according to a fourth embodiment of the present disclosure, with a fastening mechanism thereof in a catheter securing position;
Fig. 10A is a front elevational view of the catheter securement device of Fig. 9, with the fastening mechanism thereof in a position permitting catheter sliding therethrough;
Fig. 10B is a cross-sectional view of the catheter securement device of Fig. 9, taken along sectional line 9-9 of Fig. 9, with the fastening mechanism thereof in the position permitting catheter sliding therethrough;
Fig. 11A is a front elevational view of the catheter securement device of Fig. 9, with the fastening mechanism thereof in a catheter securing position;
Fig. 11B is a cross-sectional view of the catheter securement device of Fig. 9, taken along sectional line 9-9 of Fig. 9, with the fastening mechanism thereof in the catheter securing position;
Fig. 12 is a perspective top and side view of a catheter securement device according to a fifth embodiment of the present disclosure, with a fastening mechanism thereof in an open position;
Fig. 13 is a perspective top and side view of the catheter securement device of Fig. 12, with the fastening mechanism thereof in a closed, catheter securing position;
Fig. 14 is a perspective top, front and side view of a catheter securement device according to a sixth embodiment of the present disclosure;
Fig. 15 is a cross-sectional view of the catheter securement device of Fig. 14, taken along sectional line 14-14 of Fig. 14, with a fastening mechanism thereof in a position permitting catheter sliding therethrough;
Fig. 16 is a cross-sectional view of the catheter securement device of Fig. 14, taken along sectional line 14-14 of Fig. 14, with the fastening mechanism thereof in a catheter securing position; and
Fig. 17 is a perspective top, front and side view of an alternative configuration of the catheter securement device of Fig. 9.

### DETAILED DESCRIPTION OF THE DISCLOSURE

Certain terminology is used in the following description for convenience only and is not limiting. The words "lower," "bottom," "upper" and "top" designate directions in the drawings to which reference is made. The words "inwardly," "outwardly," "upwardly" and "downwardly" refer to directions toward and away from, respectively, the geometric center of the catheter securement device, and designated parts thereof, in accordance with the present disclosure. Unless specifically set forth herein, the terms "a," "an" and "the" are not limited to one element, but instead should be read as meaning "at least one." The terminology includes the words noted above, derivatives thereof and words of similar import.

It should also be understood that the terms "about," "approximately," "generally," "substantially" and like terms, used herein when referring to a dimension or characteristic of a component of the disclosure, indicate that the described dimension/characteristic is not a strict boundary or parameter and does not exclude minor variations therefrom that are functionally similar. At a minimum, such references that include a numerical parameter would include variations that, using mathematical and industrial principles accepted in the art (e.g., rounding, measurement or other systematic errors, manufacturing tolerances, etc.), would not vary the least significant digit.

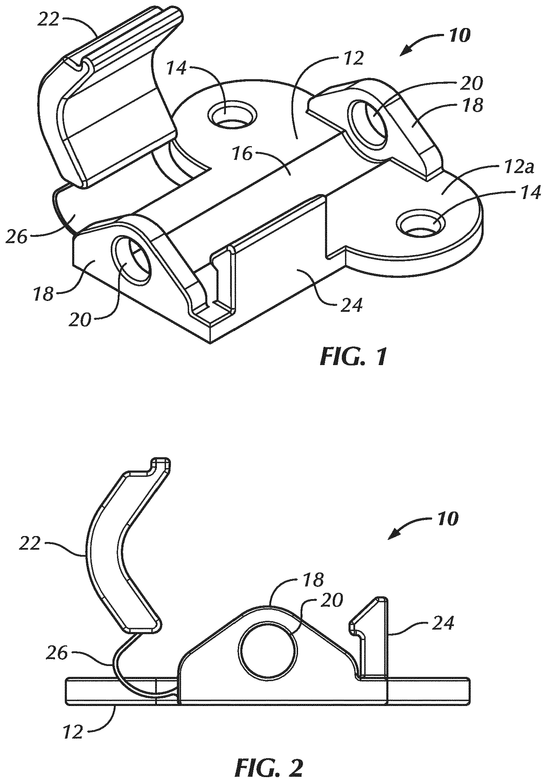

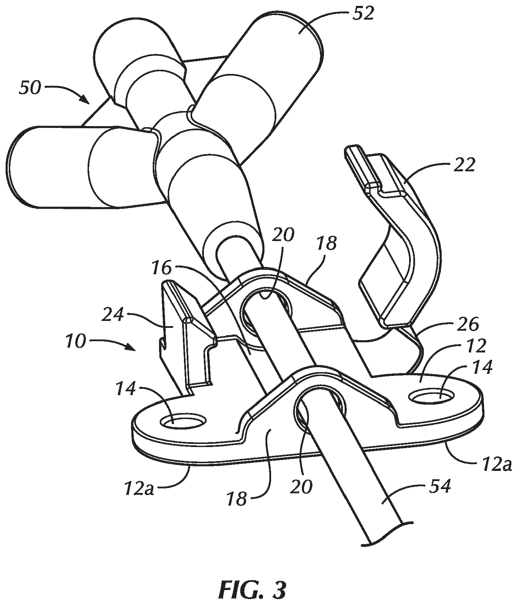

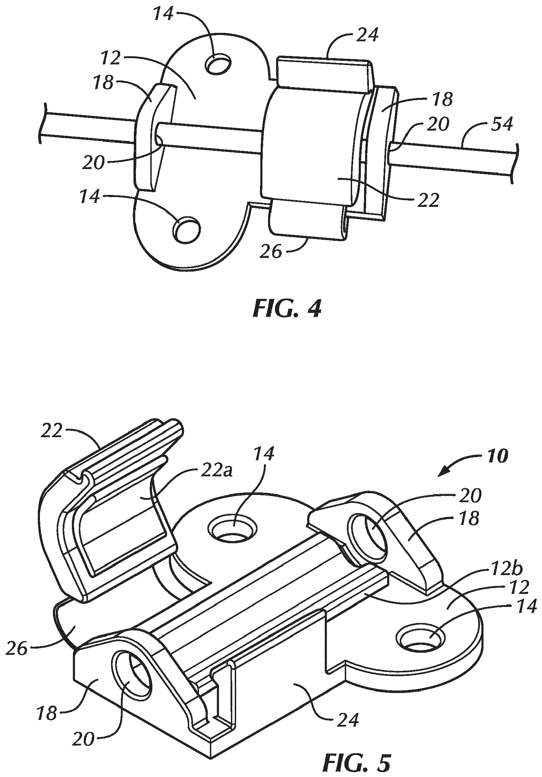

Referring to the drawings in detail, wherein like numerals indicate like elements throughout, there is shown in Figs. 1-5 a catheter securement device, generally designated 10, in accordance with a first embodiment of the present disclosure. As shown in Figs. 3, 4, the catheter securement device 10 is mounted onto a balloon occlusion catheter 50 (as will be described in further detail below), having a catheter hub 52 and a catheter shaft 54 extending therefrom, and is configured to prevent the catheter 50 from moving.

The securement device 10 includes a base surface 12 configured for placement on an intended surface, such as, for example, a patient's skin surface. The base surface 12 includes at least one fastening aperture 14, e.g., suture aperture. As will be described in further detail below, the fastening aperture(s) 14 allows for suturing the securement device 10 to a patient, thus preventing catheter 50 migration from a patient access site (not shown). In the illustrated embodiment, the base surface 12 includes two suture apertures 14, but the disclosure is not so limited. In the illustrated embodiment, the two suture apertures 14 are mirrored about a centerline of the base surface 12, within winged portions 12a of the base surface 12, but the disclosure is not so limited. As should be understood by those of ordinary skill in the art, the fastening apertures 14 may be shaped and dimensioned to permit the passage of a fastening tool, e.g., a suture, a staple or the like, therethrough.

As shown in Fig. 1, the base surface 12 may include a grooved channel 16 for stabilizing the catheter shaft 54 thereon *(see* Figs. 3, 4), but the disclosure is not so limited. In the illustrated embodiment, the channel 16 is arcuate, but the disclosure is not so limited. For example, the channel 16 may be V-shaped or the like. In the illustrated embodiment, the base surface 12 includes a pair of upwardly projecting tabs 18. As should be understood, however, the base surface 12 may include one or more than two projecting tabs 18. In the illustrated embodiment, the tabs 18 are positioned at opposing ends of the base surface 12, but the tabs 18 may alternatively be placed closer to one another (i.e., not at opposing ends of the base surface 12). Each tab 18 includes a catheter shaft aperture 20. The catheter shaft apertures 20 are dimensioned to permit substantially uninhibited sliding of the catheter shaft 54 therethrough. The catheter shaft apertures 20 are coaxial with one another and aligned with the grooved arcuate channel 16. Accordingly, as shown in Figs. 3 and 4, mounting of the securement device 10 to the catheter 50 includes passage of the catheter shaft 54 through the aperture(s) 20 and orientation of the catheter shaft 54 along the grooved channel 16. The catheter hub 52 prevents disconnection of the securement device 10 from the catheter 50 from one end and the catheter tip (not shown), e.g., an atraumatic tip, at the opposing end of the catheter 50 prevents unintentional disconnection of the securement device 10 from the catheter 50 from the other end. Advantageously, therefore, the securement device 10 remains mounted to the catheter 50, minimizing the opportunity for inadvertently losing or overlooking the securement device 10.

The securement device 10 further includes a selective fastening mechanism, e.g., a clasp, for securing the securement device 10 to the catheter 50. The fastening mechanism includes a latch 22 pivotably attached with the base surface 12 on one side of the channel 16 and a corresponding deflectable post 24 projecting upwardly from the base surface 12 at an opposing side of the channel 16. The latch 22 is movable between an open, e.g., unclasped, position (Fig. 3) and a closed, e.g., clasped, position (Fig. 4). In the open position of the latch 22, the latch 22 is disengaged from the post 24 and the securement device 10 is freely slidable along the catheter shaft 54. The deflectable post 24 is configured to form a snap engagement with the latch 22 (in a manner well understood by those of ordinary skill in the art) in the closed position of the latch 22 and the undeflected position of the post 24. In the closed position of the latch 22, e.g., engaged/clasped with the arm 24, the catheter shaft 54 may no longer move freely relative to the securement device 10, but rather they are substantially secured to one another in a non-slidable manner, e.g., via a compressive and/or friction force of the latch 22 onto the catheter shaft 54. Optionally, as shown in Fig. 5, the securement device 10 may include a compressible foam or rubberized insert 12b along the base surface 12 (positioned, e.g., along the channel 16) and/or a compressible foam or rubberized insert 22a along an underside of the latch 22, each of which provides an increased frictional force to counter sliding thereon. The insert 12b and/or the insert 22a may be configured, e.g., dimensioned, to form an interference fit with the catheter shaft 54 in the closed position of the latch 22, such that the additional frictional force(s) exhibited thereby against the catheter shaft 54 further assist(s) in preventing the catheter shaft 54 from sliding in the closed position of the latch 22.

The latch 22 may be selectively returned back to the open position thereof by temporarily deflecting (elastically) the post 24 away from the latch 22, disengaging the post 24 from the latch 22. Accordingly, the latch 22 may be selectively/repeatedly moved between the open and closed positions thereof as required. In the illustrated embodiment, the latch 22 is pivotably attached to the base surface 12 via a living hinge 26, but the disclosure is not so limited. As should be understood by those of ordinary skill in the art, the securement device 10 may be selectively secured to, i.e., substantially preventing sliding, and unsecured from, i.e., permitting substantially uninhibited sliding, the catheter shaft 54 via a variety of different methods known by those of ordinary skill in the art, such as, for example, through the use of a spring, button, strap, hardware, a combination thereof, or the like.

In use a catheter 50 is delivered to a user with the securement device 10 mounted thereto (in a manner as previously described), i.e., the securement device 10 may be mounted to the catheter 50 during manufacture thereof. With the latch 22 in the open position thereof, the securement device 10 is freely slidable along the catheter shaft 54. Accordingly, after a user, e.g., a surgeon, properly places the catheter 50 into the patient via an access site, the securement device 10 may be moved proximate to the access site. The user may then secure the securement device 10 to the patient by suturing the securement device 10 to the patient's skin surface through the suture aperture(s) 14. The user may also secure the securement device 10 to the catheter 50 by moving the latch 22 into the closed position thereof, in snapped engagement with the post 24, substantially preventing catheter 50 migration from the access site. Advantageously, if the catheter 50 requires repositioning, the sutures securing the securement device 10 to the patient may remain intact. Rather, the user unlatches the latch 22 from the post 24 (as previously described), once again permitting sliding of the catheter 50 relative to the securement device 10, and, thereafter, re-engaging the latch 22 with the post 24.

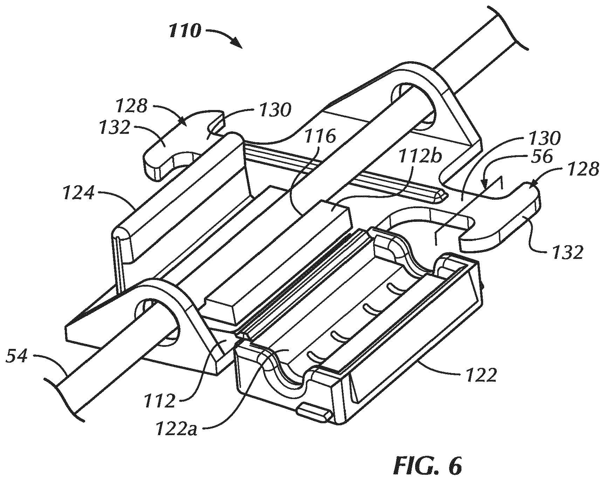

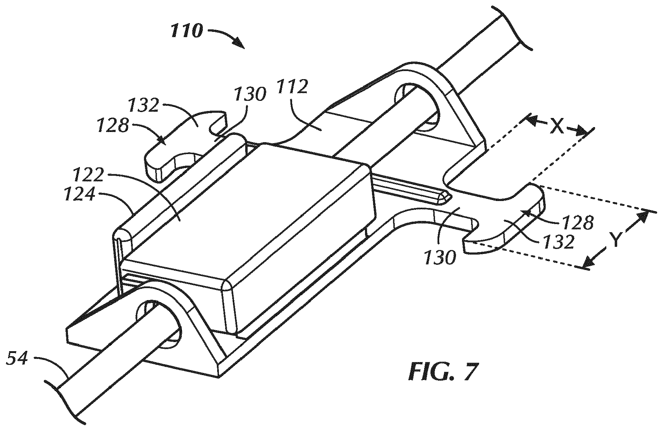

Figs. 6 and 7 illustrate a second embodiment of the catheter securement device 110. The reference numerals of the present embodiment are distinguishable from those of the above-described embodiment by a factor of one-hundred (100), but otherwise indicate the same elements as indicated above, except as otherwise specified. The catheter securement device 110 of the present embodiment is substantially similar to that of the earlier embodiment. Therefore, the description of certain similarities between the embodiments may be omitted herein for the sake of brevity and convenience, and, therefore, is not limiting.

The catheter securement device 110 includes a latch 122 and a deflectable post/wall 124 selectively engageable with, and releasable from, one another in a manner as described with respect to the securement device 10, but the disclosure is not so limited. As shown best in Fig. 6, the catheter securement device 110 may include a friction enhancing pad 112b, e.g., a compressible foam or rubberized pad or the like configured to increase frictional force to counter sliding thereon, upon the base the surface 112. The friction enhancing pad 112b may define the grooved arcuate channel 116 for stabilizing the catheter shaft 54 thereon. The latch 122 may also include an insert 122a, e.g., a compressible foam or rubberized insert or the like configured to increase frictional force to counter sliding thereon, along an underside of the latch 122, such that in the closed position of the latch 122, the catheter shaft 54 is sandwiched between the inserts 112b and 122a. In the closed position thereof, the latch 122 is also configured to substantially cover and obscure the insert 112b.

A primary difference between the catheter securement devices 10 and 110 is that the device 110 is configured to be secured to a skin surface, e.g., a patient skin surface, via skin staples 56 (rather than sutures). As shown, the base surface 112 includes at least one landing tab 128 laterally extending therefrom. In the illustrated embodiment, the landing tab 128 projects generally perpendicularly from the base surface 112, but the disclosure is not so limited, as one or more landing tabs 128 may alternatively project at a different angle from the base surface 112. In the illustrated embodiment, the base surface 112 includes a pair of oppositely disposed and oppositely directed tabs 128, but the disclosure is not so limited. For example, the base surface 112 may include more than two tabs 128. Additionally, or alternatively, the tabs 128 need not be oppositely disposed. As one example, without limitation, the tabs 128 may be constructed on opposite sides of the latch 122. Additionally, or alternatively, the tabs 128 may laterally extend in the same direction from the base surface 112.

Each tab 128 includes a neck portion 130 extending from the base surface 112 and terminating in a transverse wing portion 132, i.e., generally oriented substantially perpendicularly to the neck portion 130 (and substantially parallel with the base surface 112). A staple 56 is applied to the skin over the neck portion 130. That is, the neck portion 130 is narrowed relative to the wing portion 132 and is narrower than a width of the staple 56. The staple 56 is applied via a skin stapler (not shown) in a manner well understood by those of ordinary skill in the art. The wing portion 132 defines a length Y that may be greater than the width of the staple 56 to prevent escape of the tab 128 from underneath the staple 56. The wing portion 132 also defines a length Y smaller than the width of the stapler trench (not shown) such that the stapler may slide past the wing portion 132 and abut the base surface 112, i.e., adjacent the base of the neck portion 130, thereby providing generally consistent placement of the skin staple 56 relative to the catheter securement device 110. The neck portion defines a length X that is sufficiently long to place the wing portion 132 within the stapler trench when the stapler abuts the base surface 112.

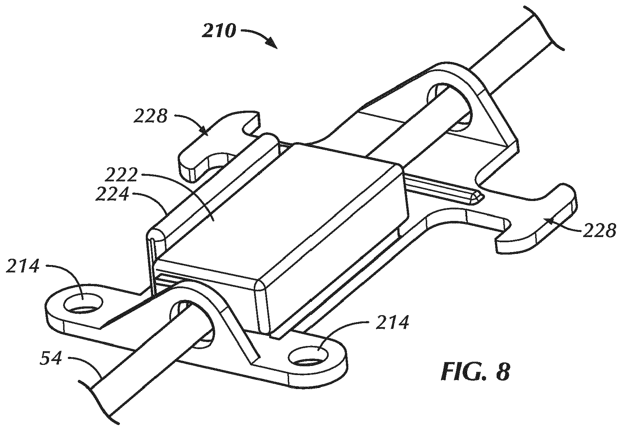

Fig. 8 illustrates a third embodiment of the catheter securement device 210. The reference numerals of the present embodiment are distinguishable from those of the above-described embodiments by a factor of two-hundred (200), but otherwise indicate the same elements as indicated above, except as otherwise specified. The catheter securement device 210 of the present embodiment is substantially similar to that of the earlier embodiments. Therefore, the description of certain similarities between the embodiments may be omitted herein for the sake of brevity and convenience, and, therefore, is not limiting.

The catheter securement device 210 employs a latch 222 and a deflectable post/wall 224 as described in securement devices 10 and 110. A primary difference between the catheter securement device 210 from the catheter securement devices 10 and 110 is that the device 210 is configured to be securable to an intended surface, e.g., a skin surface of a patient, via sutures and/or skin staples 56. That is, the catheter securement device 210 includes both at least one suture aperture 214 allowing for suturing the securement device 210 to a patient and at least one landing tab 228 allowing for stapling the securement device 210 to a patient. In the illustrated embodiment, the catheter securement device 210 includes two suture apertures 214 and two landing tabs 228, but the disclosure is not so limited. In the illustrated embodiment, the suture apertures 214 are located on an opposite side of the latch 222 from the landing tabs 228, but the disclosure is not so limited, as the suture aperture(s) 214 may be positioned on the same side of the latch 22 as the landing tab(s) 228. As should be understood, the base surface of any of the embodiments described herein may be configured as the base surface 212 in order to be securable to a patient via sutures and/or skin staples 56.

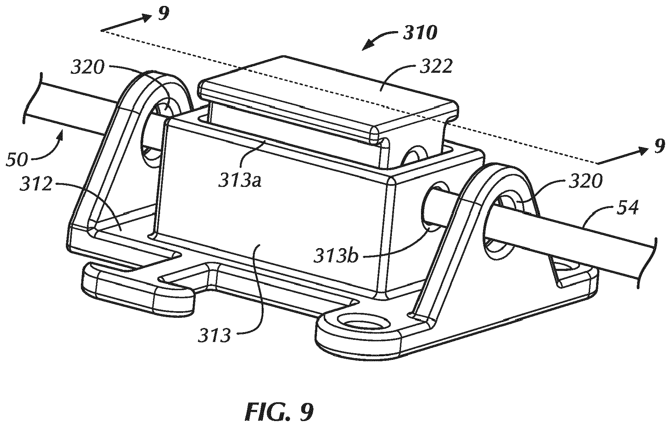

Figs. 9-11B illustrate a fourth embodiment of the catheter securement device 310. The reference numerals of the present embodiment are distinguishable from those of the above-described embodiments by a factor of three-hundred (300), but otherwise indicate the same elements as indicated above, except as otherwise specified. The catheter securement device 310 of the present embodiment is substantially similar to that of the earlier embodiments. Therefore, the description of certain similarities between the embodiments may be omitted herein for the sake of brevity and convenience, and, therefore, is not limiting.

In the illustrated embodiment, the catheter securement device 310 is configured to be securable to a patient via sutures and/or skin staples 56, as described with respect to the catheter securement device 210. As should be understood, however, the catheter securement device 310 may alternatively be configured for use with only one of sutures or skin staples 56, as described in the catheter securement devices 10 and 110, respectively.

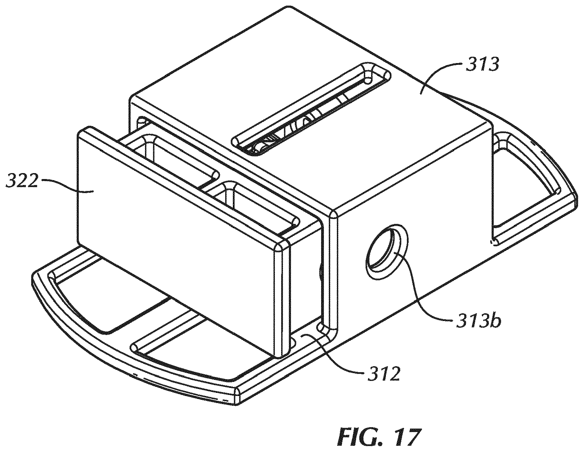

A primary difference between the catheter securement device 310 from the catheter securement devices 10, 110 and 210 is that the device 310 employs a spring biased fastening mechanism for securing the securement device 310 to the catheter 50. The fastening mechanism includes a housing 313 affixed upon the base surface 312, sized and shaped to slidably receive a plunger 322 through an open side/end of the housing 313. In the illustrated embodiment, the housing 313 is integral with, i.e., monolithic with, the base surface 312, but the disclosure is not so limited and the housing 313 may be alternatively affixed upon the base surface 312. In the illustrated embodiment, the plunger 322 is slidably received through an open upper side/end 313a of the housing 313, and translatable relative to the housing 313 in a direction substantially perpendicular to the base surface 312, as described in further detail below, but the disclosure is not so limited. The plunger 322, for example, may equally be slidably received through an open lateral side end of the housing 313 and translatable relative to the housing 313 in a direction substantially parallel to the base surface 312 *(see* Fig. 17).

The plunger 322 defines an axially extending channel 323 extending therethrough, sized and shaped to receive the catheter shaft 54 therethrough. The housing 313 defines a pair of oppositely disposed apertures 313b, e.g., in the respective front and rear surfaces thereof, that are axially aligned with the catheter shaft apertures 320 in the respective tabs 318. A spring or other biasing member 327, e.g., a coil spring or the like, is positioned between the movable plunger 322 and a stationary surface of the securement device 310, e.g., a surface opposing the open side/end of the housing 313. In the illustrated embodiment, the spring 327 is positioned between an underside of the plunger 322 and the base surface 312 within the housing 313, but the disclosure is not so limited. For example, the spring 327 may be positioned between the plunger 322 and a closed end of the housing 313. The spring 327 is configured to provide an ejecting force biasing the plunger 322 through the open side/end of the housing 313, causing misalignment between the channel 323 and the apertures 313b. In the illustrated embodiment, the spring 327 applies an upwardly directed biasing force on the plunger 322. The plunger 322, however, is configured to remain mechanically interconnected with the housing 313 despite the biasing force of the spring 327 in a manner well understood by those of ordinary skill in the art. For example, without disclosure, the plunger 322 may include a surface *(see,* e.g., Fig. 17), e.g., a tab or rib, engageable with a shoulder or other stop surface *(see,* e.g., Fig. 17) of the housing 313 to define a maximum displacement of the plunger 322 relative to the housing 313 and prevent the ejecting force of the spring 327 from separating the plunger 322 from the housing 313.

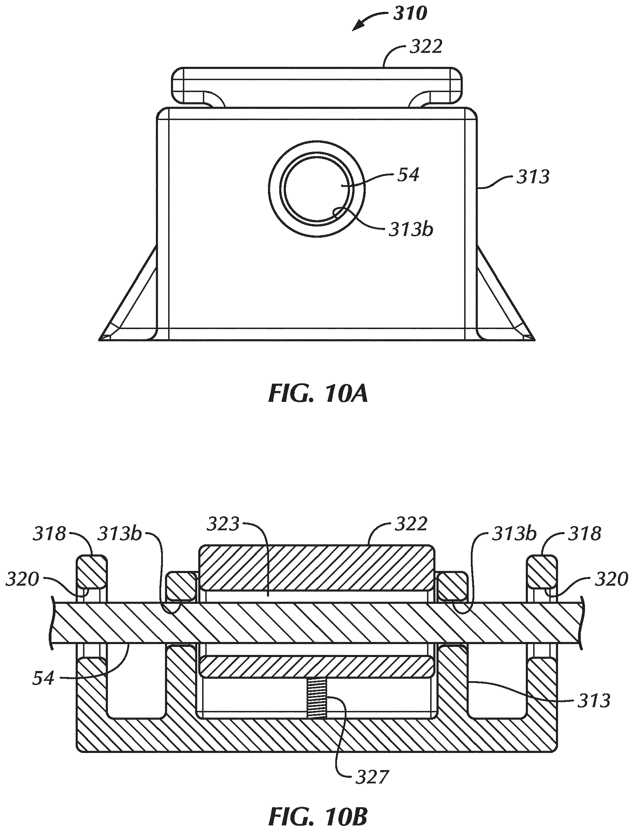

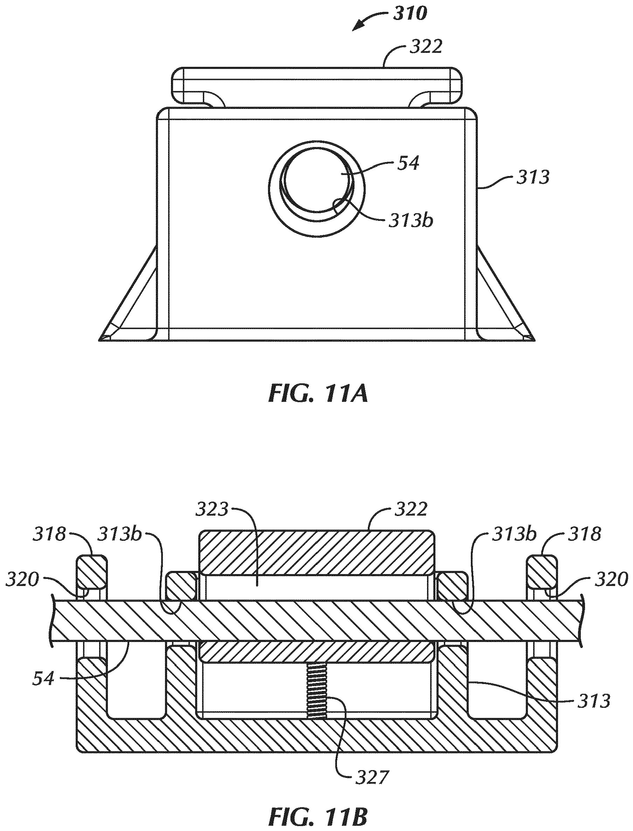

As shown in Figs. 10A and 10B, the plunger 322 is actuatable, e.g., depressible, in a direction opposing the biasing force of the spring 327 (and by applying a force greater than the biasing force), to axially align the channel 323 with, and between, the apertures 313b of the housing 313 and the catheter shaft apertures 320 of the respective tabs 318. In such an aligned position, the catheter shaft 54 is slidable and extendible through the respectively opposing pairs of apertures 320 and 313b and through the intervening channel 323 axially aligned therewith. To secure the device 310 to the catheter 50, the plunger 322 is subsequently released, such that the biasing force of the spring 327 automatically drives the plunger 322 in a direction away from the housing 313 and, in turn, secures and/or pins the catheter shaft 54 between the interior sidewall of the apertures 313b and the interior sidewall of the channel 323 (*see* Figs. 11A, 11B) to substantially prevent sliding of the catheter shaft 54 relative to the device 310. That is, a constant clamping force is applied onto the catheter shaft 54 by the plunger 322 to substantially secure the shaft 54 against sliding. As should be understood, the plunger 322 may be selectively depressed as needed by a user to free the catheter shaft 54 to slide relative to the securement device 310.

Advantageously, the fastening mechanism of the catheter securement device 310, i.e., the spring biased plunger, is compatible for use with multiple differently sized catheters 50. That is, the apertures 320, 313b as well as the channel 323 may be dimensioned to accommodate a large diameter catheter shaft therethrough but may generally also be utilized with any smaller diameter catheter shafts as the spring 327 will bias the plunger 322 until the catheter is clamped between the apertures 313b and the channel 323, irrespective of the diameter of the catheter shaft 54. Optionally, the channel 323 may include, e.g., be lined with, a friction enhancing liner/insert, e.g., a compressible foam or rubberized insert or the like, to provide an increased frictional force upon the catheter shaft 54 to counter sliding thereon and also may operate as padding between the catheter shaft 54 and the channel 323.

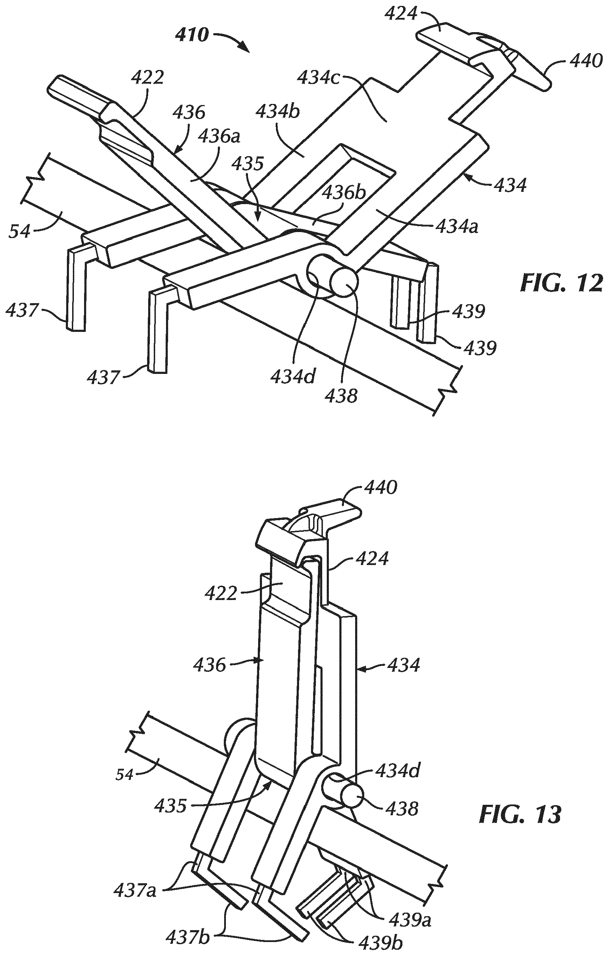

Figs. 12 and 13 illustrate a fifth embodiment of the catheter securement device 410. The reference numerals of the present embodiment are distinguishable from those of the above-described embodiments by a factor of four-hundred (400), but otherwise indicate the same elements as indicated above, except as otherwise specified. The catheter securement device 410 of the present embodiment is substantially similar to that of the earlier embodiments. Therefore, the description of certain similarities between the embodiments may be omitted herein for the sake of brevity and convenience, and, therefore, is not limiting.

A primary difference of the catheter securement device 410 from the catheter securement devices 10, 110, 210 and 310 is that the device 410 is configured to secure the catheter 50 against the skin without requiring an external securement component, i.e., suture or staple. The securement device 410 includes first and second legs 434, 436 pivotably coupled with one another about a fulcrum 435. The first leg 434 defines a main body portion in the form of a clevis. That is, the first leg 434 includes a pair of parallel and spaced apart arms 434a and 434b connected with one another by a top traverse section 434c. The arms 434a, 434b each include a respective through-bore 434d, the through-bores 434d being aligned with one another. In the illustrated embodiment, the arms 434a and 434b are curvilinear about the through-bores 434d. That is, the section of the arm 434a, 434b proximal to the through bore 434d is angled, e.g., an obtuse included angle, with respect to the section of the arm 434a, 43b distal of the through-bore 434d.

Each arm 434a, 434b includes a finger 437 extending from an end thereof opposite the top transverse section 434c. Each finger 437 is generally hook or L-shaped. That is, each finger 437 includes a first section 437a and a second section 437b that are angularly connected with one another. In the illustrated embodiment, the fingers 437 are oriented substantially parallel with one another, but the disclosure is not so limited.

The second leg 436 includes an elongate body 436a which projects through the space between the arms 434a and 434b of the first leg 434. The body 436a includes a channel 436b extending therethrough and aligned between the through-bores 434d. A pin 438 extends through the through-bores 434d and the intervening channel 436b to form the fulcrum 435 and the axis about which the first and second legs 434, 436 are pivotable relative to one another. In the illustrated embodiment, the body 436a is also curvilinear about the channel 436b. That is, the section of the body 436a proximal to the channel 436b is angled, e.g., an obtuse included angle, with respect to the section of the body 436a distal of the channel 436b.

A pair of fingers 439 extend from an end of the body 436a proximate the fingers 437. Similarly to the fingers 437, each finger 439 is also generally hook or L-shaped. That is, each finger 439 includes a first section 439a and a second section 439b that are angularly connected with one another. In the illustrated embodiment, the fingers 437 are oriented substantially parallel with one another, but the disclosure is not so limited.

The first and second legs 434, 436 are configured to releasably engage one another via a clasp mechanism formed at the proximal ends thereof. The first leg 434 includes a hook shaped post 424 proximally projecting from the top transverse section 434c, and the second leg 436 includes a proximally projecting latch 422. The legs 434, 436 are movable toward once another between an open, e.g., unclasped, position (Fig. 12) and a closed, e.g., clasped, position (Fig. 13). In the open position of the securement device 410, the latch 422 is disengaged from the hook-shaped post 424 and the catheter shaft 54 is not secured by the securement device 410. The hook-shaped post 424 is configured to form a secure, snap/latch engagement with the latch 422 (in a manner well understood by those of ordinary skill in the art) in the closed position of securement device 410.

In use, the catheter shaft 54 is placed between the first and second legs 434, 436, underneath the fulcrum 435, when the securement device 410 is in the open position thereof. The securement device 410 is subsequently placed upon the intended surface, e.g., the skin surface of a patient, and the legs 434, 436 are pivoted toward one another to engage the latch 422 with the post 424. Movement of the device 410 into the closed position thereof clamps the first and second legs 434, 436 around the catheter shaft 54 such that the catheter shaft 54 may no longer move freely relative to the securement device 410, but rather they are substantially secured to one another in a non-slidable manner. Advantageously, and similarly to the catheter securement device 310, the securement device 410 is compatible for use with multiple differently sized catheters 50. That is, the legs 434, 436 are selectively pivotable relative to one another and relative to an intervening catheter shaft 54 are appropriate until the legs clamp the catheter shaft 54 therebetween, and, therefore, may accommodate for multiple differently sized catheters 50.

Movement of the device 410 into the closed position also moves the fingers 437, 439 toward one another to pinch the intended surface therebetween or pierce through the intended surface to stabilize the securement device 410 and the catheter shaft 54 relative to the intended surface. That is, the finger 437, 439 may operate as built-in staples of the securement device 410.

The hook-shaped post 424 includes a tab 440 at a top end thereof that laterally extends therefrom. The tab 440 is selectively depressible to deflect the post 424 out of engagement with, and release, the latch 422. Accordingly, the securement device 410 is selectively releasable from the intended surface as well as from the catheter shaft 54 when desired.

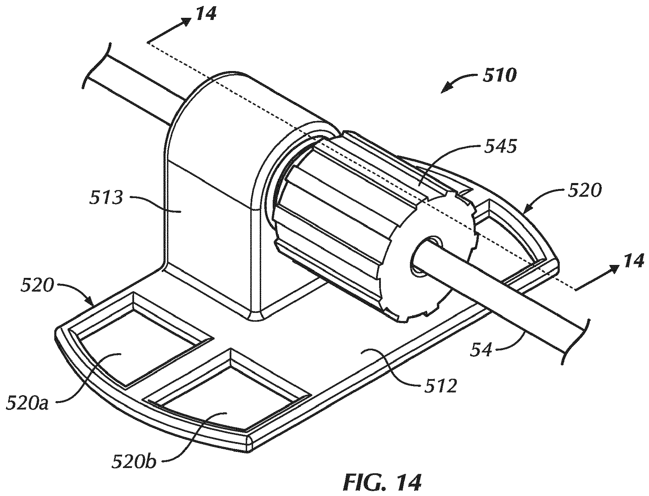

Figs. 14-16 illustrate a sixth embodiment of the catheter securement device 510. The reference numerals of the present embodiment are distinguishable from those of the above-described embodiments by a factor of four-hundred (400), but otherwise indicate the same elements as indicated above, except as otherwise specified. The catheter securement device 510 of the present embodiment is substantially similar to that of the earlier embodiments. Therefore, the description of certain similarities between the embodiments may be omitted herein for the sake of brevity and convenience, and, therefore, is not limiting.

A primary difference of the catheter securement device 510 from the catheter securement devices 10, 110, 210, 310 and 410 is that the device 510 is configured to secure the catheter 50 via a screw-type fastening mechanism. As shown, the securement device 510 includes a housing 513 affixed upon the base surface 512, having an externally threaded screw 542, e.g., a female Luer, axially projecting therefrom. The housing 513 includes a first axially extending channel 523 extending therethrough and the screw 542 includes a second axially channel 543 extending therethrough. The first and second axially extending channels 523, 543 are axially aligned and positioned adjacent one another in series, and sized and shaped to receive the catheter shaft 54 therethrough. The second axially extending channel 543 is larger/wider than the first axially extending channel 523, forming a shoulder 543a therebetween. A compressible member 544, e.g., an O-ring or other compressible friction bearing type member, constructed of a polymer or other compressible material with static friction properties, is fitted within the second axially extending channel 543, abutting the shoulder 543a. An internally threaded collar 545, e.g., a male Luer, is threadedly mounted upon the externally threaded screw 542. The collar 545 includes an axially oriented inner tubular body 546 (being round, square or other shaped in cross-section) extending within the second axially extended channel 543. The inner tubular body 546 defines a third axially extending channel 546a extending therethrough, axially aligned with the first and third channels 523, 543 and also sized and shaped to receive the catheter shaft 54 therethrough.

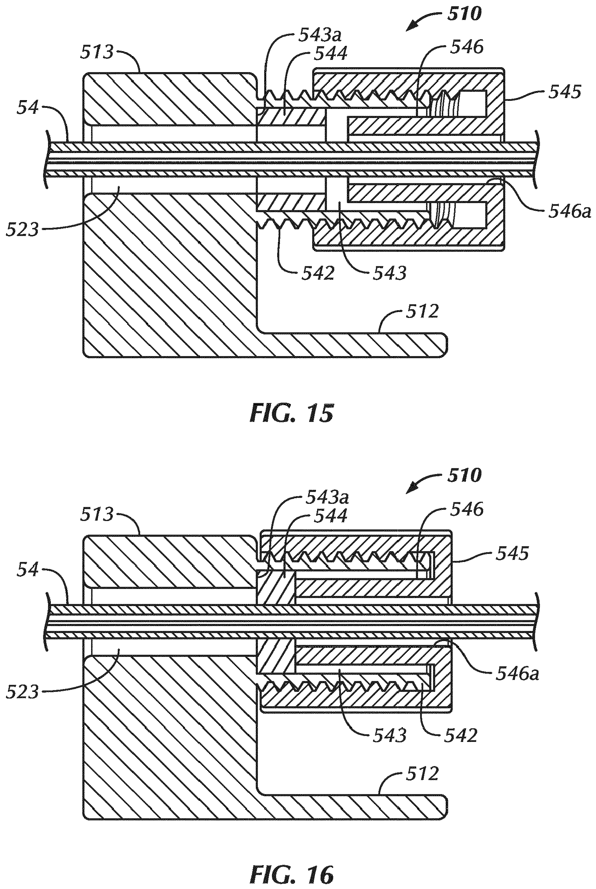

In use, the collar 545 is sufficiently unscrewed from the screw 542, such that the inner tubular body 546 is substantially not engaged with the compressible member 544 *(see* Fig. 15). The catheter shaft 54 is substantially freely advanced through the first, second and third channels 523, 543, 546a and subsequently the collar 545 is threaded further upon the screw 542 such that the inner tubular body 546 engages and compresses the compressible member 544. The compressible member 544 is, therefore, fixed between the periphery of the second channel 543, the shoulder 543a and the inner tubular body 546. Further threading of the collar 545, therefore, radially compresses the compressible member 544 *(see* Fig 16) to, in turn, engage and secure the catheter shaft 54 against sliding movement relative thereto. Optionally, a stop surface (not shown) may be employed to prevent over-threading of the collar 545, and, therefore, prevent over-tightening of the compressible member 544 upon the shaft 54 to prevent catheter shaft 54 damage. The collar 545 is selectively unthreaded from the screw 542 as needed by a user to re-enable catheter shaft 54 sliding relative to the securement device 510.

Advantageously, the fastening mechanism of the catheter securement device 510, i.e., the screw-type fastening mechanism, is compatible for use with multiple differently sized catheters 50. That is, the axially extending first, second and third channels 523, 543, 546a may be dimensioned to accommodate a large diameter catheter shaft therethrough but may generally also be utilized with any smaller diameter catheter shafts as the collar 545 may be threaded upon the screw 542 as necessary to sufficiently compress the compressible member 544 upon the catheter 50, irrespective of the diameter of the catheter shaft 54.

Turning to the base surface 512 of the catheter securement device 510, and as shown in Fig 14, at least a portion of the base surface 512 includes a window-like lattice 520 including a plurality of windows frames 520a defining windows 520b. Accordingly, a user may selectively secure the securement device 510 to an intended surface, e.g., a skin surface of a patient, via sutures and/or skin staples 56. For example, a user may apply a skin staple across two windows 529b, with an intervening window frame 520a structure. Alternatively, a user may suture the securement device 510 to the intended surface through a single window 529b and about the window frame 520a structure thereof. In the illustrated embodiment, the catheter securement device 510 include two opposing window-like lattices 520 on opposite sides of the housing 513, but the disclosure is not so limited. In the illustrated embodiment, each window-like lattice 520 includes two windows 529b, but the disclosure is not limited.

It will be appreciated by those skilled in the art that changes could be made to the embodiments described above without departing from the broad inventive concept thereof. For example, for the securement device to stay permanently attached to the catheter 50, it could be molded in two parts, completely enclosing the catheter shaft 54. The securement device could also be attached to the catheter 50 via a wire or thread. It is understood, therefore, that this disclosure is not limited to the particular embodiments disclosed, but it is intended to cover modifications within the spirit and scope of the present disclosure, as set forth in the appended claims.

## Claims

1. A catheter and a catheter securing device kit comprising:
a catheter having a catheter shaft; and
a catheter securing device comprising:
a base surface selectively securable to an intended surface;
at least one catheter aperture, the catheter shaft extending through the at least one catheter aperture, such that the catheter securing device is mounted on the catheter shaft;
a channel for stabilizing the catheter shaft thereon; and
a fastener repeatedly movable between a first position and a second position, independently of securement of the base surface to the intended surface, wherein, in the first position, the fastener permits substantially free sliding of the catheter shaft through the at least one catheter aperture, and, in the second position, the fastener substantially secures the catheter shaft to the catheter securing device in a substantially non-sliding manner.

2. The kit of claim 1, wherein the fastener comprises:
a deflectable post projecting from the base surface; and
a latch selectively movable relative to the base surface,
wherein, in the first position, the latch is disengaged from the deflectable post, and in the second closed position, the latch is in snap engagement with the deflectable post, substantially securing the catheter shaft to the catheter securing device via at least one of a compressive or friction force of the latch onto the catheter shaft.

3. The kit of claim 2, wherein the deflectable post is positioned on one side of the channel and the latch is pivotably attached to the base surface at an opposing side of the channel, whereby, in the second position, the latch extends over the channel.

4. The kit of claim 2, wherein the deflectable post is selectively and elastically deflectable to disengage the latch therefrom and return the latch from the second position into the first position thereof.

5. The kit of claim 2, wherein the catheter securement device further comprises at least one of a friction enhancing insert along the channel or a friction enhancing insert along an underside of the latch, configured to form an interference fit with the catheter shaft in the closed position of the latch.

6. The kit of any one of the previous claims, wherein the catheter securement device further comprises a friction enhancing pad upon the base surface, the channel being formed in the friction enhancing pad.

7. The kit of claim 1, wherein the fastener comprises:
a housing upon the base surface, the housing having an open side and a pair of oppositely disposed apertures formed in opposing peripheral sides of the housing;
a plunger slidably received within the housing via the open side thereof, the plunger defining the channel; and
a biasing member positioned between the plunger and a surface opposing the open side of the housing, the biasing member being configured to provide an ejecting force biasing the plunger through the open side of the housing;
the catheter shaft extending through the at least one catheter aperture, the pair of oppositely disposed apertures of the housing and the at least one catheter aperture, wherein, in the second position, the ejecting force misaligns the channel from the pair of oppositely disposed apertures of the housing and the at least one catheter aperture, thereby securing the catheter shaft between the channel and the pair of oppositely disposed apertures of the housing, and in the first position, the channel being substantially aligned with the pair of oppositely disposed apertures of the housing and the at least catheter aperture, permitting substantially free sliding of the catheter shaft therethrough.

8. The kit of claim 7, wherein the ejecting force is configured to automatically position the fastener in the second position thereof, the fastener being selectively movable to the first position thereof under application of a force opposing, and greater than, the ejecting force.

9. The kit of claim 7. wherein the catheter securement device further comprises a friction enhancing insert along the channel.

10. The kit of claim 1, wherein the fastener comprises:
a housing upon the base surface having an externally threaded screw projecting therefrom, the housing and externally threaded screw defining the channel;
a compressible member positioned along the channel, the catheter shaft extending along the channel and through the compressible member; and
an internally threaded collar selectively threadably advanceable and retractable upon the externally threaded screw;
wherein, in the second position, the collar is threadedly advanced upon the externally threaded screw to a position radially compressing the compressible member upon the catheter shaft to substantially prevent sliding of the catheter shaft therethrough along the channel, and in the first position, the collar is threadedly retracted from the externally threaded screw relative to the second position to a position wherein the compressible member permits substantially free sliding of the catheter shaft therethrough and along the channel.

11. The kit of any one of the previous claims, wherein the catheter securement device further comprises at least one upwardly projecting tab, each one of the at least one upwardly projecting tab including one of the at least one catheter aperture.

12. The kit of any one of the previous claims, wherein the catheter securement device further comprises at least one of a fastening aperture or a landing tab, the fastening aperture being positioned along the base surface and configured for receiving a fastening tool therethrough to secure the catheter securement device to the intended surface, and the landing tab comprising a neck portion extending laterally from the base surface and terminating in a transverse wing portion, the neck portion being narrowed relative to the wing portion and dimensioned to permit application of a staple thereupon.

13. The kit of any one of claims 1-11, wherein the base surface further comprises a pair of oppositely disposed, laterally projecting winged portions, each of the winged portions having a fastening aperture therein configured for receiving a fastening tool therethrough to secure the catheter securement device to the intended surface.

14. The kit of any one of claims 1-11, wherein the base surface further comprises at least one landing tab, the landing tab comprising a neck portion extending laterally from the base surface and terminating in a transverse wing portion, the neck portion being narrowed relative to the wing portion and dimensioned to permit application of a staple thereupon.

15. The kit of any one of the claims 1-11, wherein at least a portion of the base surface comprises a window-like lattice defined by a plurality of window frames forming windows therebetween, the window-like lattice being configured to selectively secure the catheter securing device to the intended surface via a fastening tool.
